# EUROPÄISCHE PATENTANMELDUNG

(11) **EP 4 014 854 A1**
(43) Veröffentlichungstag der Anmeldung: **22.06.2022**
(21) Anmeldenummer: 20215557.8
(22) Anmeldetag: 18.12.2020
(51) Int. Cl.: A61B 5/0531, A61B 5/00, A61B 5/257, A61B 5/28, A61B 5/291, A61B 5/01

(54) **SENSOREINHEIT ZUR BEFESTIGUNG AN EINEM MENSCHLICHEN ODER TIERISCHEN KÖRPER**

(71) Anmelder: InnoME GmbH, 32339 Espelkamp (DE); Covestro Deutschland AG, 51373 Leverkusen (DE)
(72) Erfinder: Weiser, Marc-Stephan, 51515 Kürten (DE); Hüttner, Martin, 51375 Leverkusen (DE); Kossel, Klas-Moritz, 27283 Verden (DE); Ahlswede, Marco, 29683 Bad Fallingbostel (DE); Krämer, Thorsten, 40764 Langenfeld (DE); Baasner, Daniel, 32427 Minden (DE); Muth, Bjarne, 32312 Lübbecke (DE); Kottkamp. eike Wilhelm, 32312 Lübbecke (DE)
(74) Vertreter: Aulich, Martin

(57) **Zusammenfassung**

Die Erfindung betrifft eine Sensoreinheit zur Befestigung an einem menschlichen oder tierischen Körper, insbesondere an der Haut des Körpers, mit einem Sensorteil (11), das einen Foliensensor (12) zur Messung ein oder mehrerer Körperparameter aufweist, der über ein Folienstück (14) mit mindestens einer an dem Folienstück (14) angeordneten elektrischen Leitung verfügt sowie über einen mit dieser Leitung elektrisch leitend verbundenen, bevorzugt an dem Folienstück (14) angeordneten Messfühler (13), und mit einem mit dem Sensorteil (11) bevorzugt lösbar verbundenen Elektronikteil (17), das eine Elektronikeinheit zum Betreiben des Foliensensors aufweist.

## Beschreibung

Die vorliegende Erfindung betrifft eine Sensoreinheit zur Befestigung an einem menschlichen oder tierischen Körper, insbesondere an der Haut des Körpers, sowie ein Sensorteil und ein Elektronikteil für eine solche Sensoreinheit.

Es existieren verschiedene Sensoreinheiten am Markt, die zur unmittelbaren Befestigung am menschlichen Körper vorgesehen sind und mit denen verschiedene Körperparameter erfasst werden können, wie etwa die elektrischen Aktivitäten des Herzens oder des Gehirns.

In der Regel verfügen diese Sensoreinheiten - neben ein oder mehreren geeigneten Sensorelementen - über eine eine Energieversorgung umfassende Elektronikeinheit, mit der das jeweilige Sensorelement betrieben wird und die die Messergebnisse speichern und/oder auswerten kann.

Die bekannten Lösungen bauen in der Regel stark auf, da die Elektronikeinheit zum Schutz vor Umgebungseinflüssen in einen starren Kunststoffbehälter eingehaust werden muss bzw. wird, sodass sie von der jeweiligen Person, an deren Körper die Sensoreinheit befestigt wird, als störend empfunden werden.

Zudem kann es bei verschiedenen Lösungen zu Hygieneproblemen kommen, wenn die Sensoreinheiten und insbesondere deren hautberührenden oder hautnahen Sensorelemente nacheinander für unterschiedliche Personen eingesetzt werden.

Ausgehend hiervon ist es Aufgabe der vorliegenden Erfindung, Sensoreinheiten, die dafür ausgebildet und vorgesehen sind, an menschlichen oder tierischen Körpern befestigt zu werden, weiterzuentwickeln.

Diese Aufgabe wird gelöst durch eine Sensoreinheit mit den Merkmalen des Anspruchs 1 bzw. des Ausführungsbeispiels 1, ein Sensorteil für eine solche Sensoreinheit mit den Merkmalen des Anspruchs 15 bzw. des Ausführungsbeispiels 26 sowie ein Elektronikteil für eine solche Sensoreinheit mit den Merkmalen des Anspruchs 16 bzw. des Ausführungsbeispiels 28.

Eine erfindungsgemäße Sensoreinheit zur Befestigung an einem menschlichen oder tierischen Körper, insbesondere an der Haut des Körpers, umfasst dementsprechend ein Sensorteil, das einen Foliensensor zur Messung ein oder mehrerer Körperparameter aufweist, wobei der Foliensensor über ein vorzugsweise flexibles Folienstück mit mindestens einer an dem Folienstück angeordneten elektrischen Leitung verfügt sowie über ein mit dieser elektrisch leitend verbundenes, bevorzugt an dem Folienstück angeordneter Messfühler. Weiter umfasst die Sensoreinheit ein mit dem Sensorteil bevorzugt lösbar verbundenes Elektronikteil, das eine Elektronikeinheit zum Betreiben des Foliensensors aufweist.

Was die Befestigung der Sensoreinheit an dem Körper betrifft, so kann die Sensoreinheit bevorzugt mit der Haut des Körpers verklebt sein. Insbesondere kann dabei der Foliensensor, vorzugsweise das Folienstück des Foliensensors (mindestens jeweils ein Teil desselben), mit der Haut verklebt sein.

Als Material für die Verklebung der Sensoreinheit bzw. des Foliensensors, insbesondere des Folienstücks, auf der Haut des Körpers kann jedes Material eingesetzt werden, das der Fachmann hierfür auswählen würde. Bevorzugt umfasst das Material zur Verklebung einen nicht zytotoxischen Klebstoff oder ist als solcher ausgebildet. Der Klebstoff ist bevorzugt ausgewählt aus der Gruppe bestehend aus Acrylklebstoff, ein (thermoplastischer) Polyurethanklebstoff, ein Epoxidharz-Klebstoff, ein Silikonklebstoff, ein Kautschukklebstoff oder eine Mischung aus mindestens zwei hiervon. Bevorzugt wird einer der Polyurethanklebstoffe verwendet wie er in den Publikationen zu EP 3 502 156 A1 oder US 2019/0233691 A1 beschrieben ist.

Bevorzugt ist es, in oder für die Sensoreinheit, insbesondere für oder als das flexible Folienstück, eine flexible, elastische Folie einzusetzen. Die Folie weist bevorzugt ein Polymer ausgewählt aus der Gruppe bestehend aus Polyethylen, weiches Polyethylen (LDPE), Polypropylen, Polyethylenterephthalat, Polybutylenterephthalat, Polyurethan, thermoplastisches Polyurethan oder eine Mischung oder Kombination aus mindestens zwei hiervon auf.

Die Folie weist bevorzugt eine Durchlässigkeit gegenüber Wasserdampf in einem Bereich von 2 bis 100 g/24 h * m², bevorzugt 3 bis 50 g/24 h * m², besonders bevorzugt von 5 bis 20 g/24 h * m² (Bestimmung nach DIN 53122-1-2001 bei Klima B (38°C und 90 % relativer Luftfeuchte) und wurde an 150 µm Folien gemessen, wenn nichts anderes gefordert wurde) auf.

Es ist bevorzugt, die Folie, beispielsweise in Form einer Membranfolie, als Wasserdampfbarriere einzusetzen. Die Folie sollte neben einer geringen Durchlässigkeit für Wasser und Wasserdampf ein mechanisches Eigenschaftsprofil aufweisen, das sie für den Einsatz in der Sensoreinheit eignet, die sowohl mechanischen Belastungen durch die Bewegung des Menschen oder Tieres ausgesetzt ist als auch durch ein eventuelles Auseinander- und Zusammensetzen der Sensoreinheit. Bevorzugt ist die Folie schweißbar, kälteflexibel und beständig gegen Tenside oder andere Medieneinflüsse, wie sie im täglichen Leben eines Menschen oder Tieres auftreten. Vorzugsweise sollte die Folie antistatisch ausrüstbar bzw. ausgerüstet sein.

Diese vorgenannten Eigenschaften der Folie, insbesondere der Membranfolie können bevorzugt dadurch erreicht werden, dass die Folie ein- oder mehrschichtig aufgebaut ist, wobei bevorzugt mindestens eine Schicht ein thermoplastisches Polyurethan (TPU) aufweist oder daraus besteht.

Erfindungsgemäß bevorzugt werden TPU eingesetzt, deren längerkettige Diolkomponente ein Polyester oder Polyether ist, und die eine Shore-Härte von vorzugsweise 75 - 95 A, besonders bevorzugt 85 - 95 A, bestimmt nach DIN 53 505, aufweisen. Besonders bevorzugt sind TPU mit Polyether-Diolkomponente (TPU-Ether).

Bevorzugt ist die Kombination von TPU-Ether- und TPU-Ester-Schichten in einer Mehrschichtfolie.

Geeignete thermoplastische Polyurethane sind beispielsweise unter den Handelsnamen Desmopan^{®} und Texin^{®} (Bayer MaterialScience AG, Leverkusen, DE), Elastollan^{®} (Elastogran GmbH, Lemförde, DE), Estane^{®} (Lubrizol Advanced Materials Inc., Cleveland, OH, USA) oder Morthane^{®} (Morton International, Inc., Chicago, III, USA) erhältlich.

Bevorzugt weist die Folie in mindestens einer Schicht, insbesondere in der aus thermoplastischen Polyurethanen gebildeten Schicht, zusätzlich gebräuchliche Additive auf, die aus der Gruppe umfassend
I. Antiblockmittel, anorganische oder organische Abstandshalter,
II. Gleit- oder Entformungsmittel,
III. Pigmente oder Füllstoffe und
IV. Stabilisatoren
oder einer Kombination aus mindestens zwei hiervon ausgewählt werden.

Der Anteil der genannten Additive I bis IV liegt in Summe bevorzugt zwischen 0 Gew.-% und 30 Gew.-%.

Bevorzugt enthält die äußere Schicht, insbesondere die TPU-Schicht bis zu 30 Gew.-% eines Antistatikums.

Additive, die in den bevorzugten Folien enthalten sind, sind beispielsweise bei Gächter und Müller beschrieben in: Plastics Additives, Carl Hanser Verlag München, 4. Ausgabe (1996).

Ein mindestens dreischichtiger Aufbau einer Mehrschichtfolie ist bevorzugt dadurch gekennzeichnet, dass mindestens eine Schicht auf Basis eines Polyethylens (PE), bevorzugt eines weichen Polyethylens, auch "low-density" Polyethylen (LDPE) genannt, zwischen zwei aus thermoplastischem Polyurethan aufgebauten Schichten liegt.

Geeignete Polyethylene weisen bevorzugt eine Dichte in einem Bereich von 0,915 bis 0,935 g/l auf. Bevorzugt weist das Polyethylen eine mittlere Molekulare Masse in einem Bereich von 5000 bis 20 000 g/mol, weiter bevorzugt in einem Bereich von 7000 bis 15 000 g/mol auf.

Weiterhin bevorzugt weist die Folie einen mindestens fünfschichtigen Aufbau auf, wobei TPU in den beiden Außenschichten vorliegt. Bevorzugt ist eine Mittelschicht auf Basis eines Polyethylens vorgesehen, die über Zwischenschichten, sog. Haftvermittlerschichten, die aus einem Copolymer-basierenden Haftvermittler gebildet werden, mit den beiden TPU Außenschichten verbunden ist.

Copolymer-basierende Haftvermittler sind durch Abstufungen sowohl polarer als auch unpolarer Molekülbestandteile gekennzeichnet. Besonders bevorzugt sind Olefin-basierende Polymer-Haftvermittler.

Zu der Gruppe der Olefin-basierenden Polymer-Haftvermittler gehören insbesondere Ethylen-Vinylacetat-Copolymere und Ethylen-Acrylat-Copolymere mit einem Comonomer-Anteil niedriger als der Olefin-Anteil. Besonders bevorzugt sind Olefin-basierende Copolymere mit Comonomer-Anteilen von 15 bis 30 Gew.-%.

Bevorzugt sind Folien mit einer Gesamtdicke in einem Bereich von ≥ 50 bis ≤ 1000 µm, weiter bevorzugt von ≥ 70 bis ≤ 900 µm, besonders bevorzugt von ≥ 90 bis ≤ 800 µm.

Bei einer mehrschichtigen Folie liegen die Dicke der Einzelschichten aus thermoplastischen Polyurethanen bevorzugt in einem Bereich von ≥ 20 bis ≤ 350 µm, die Dicke der Polyethylen-Schicht in einem Bereich von ≥ 5 bis ≤ 50 µm und die der Haftvermittler-Schichten bevorzugt in einem Bereich von ≥ 5 bis ≤ 200 µm.

Bevorzugt besteht die Folie aus mindestens zwei außen liegenden Schichten TPU und einer innen liegenden Schicht Polyethylen, insbesondere weichem Polyethylen.

Zur Herstellung der Ein- oder Mehrschichtfolie eignen sich besonders die gängigen thermischen Umformverfahren zur Verarbeitung von Kunststoffen zu ein- oder mehrschichtigen Flächengebilden. Hier wäre die Herstellung durch (Co-)Extrusion zu nennen, die bevorzugt nach dem Blasfolienverfahren erfolgt. Aufgrund der besseren erzielbaren Verbundhaftung ist die Coextrusion unter den geeigneten Herstellungsverfahren von mehrschichtigen thermoplastischen Flächengebilden im besonderen Maß bevorzugt.

Die im Rahmen der nachfolgenden Beispiele und Vergleichsbeispiele beschriebenen Folien wurden durch Blasfoliencoextrusion hergestellt. Die zum Aufschluss thermoplastischer Harze geeigneten Schneckenwerkzeuge sind in ihrem Aufbau z.B. von Wortberg, Mahlke und Effen in: Kunststoffe, 84 (1994) 1131-1138, von Pearson in: Mechanics of Polymer Processing, Elsevier Publishers, New York, 1985 oder der Fa. Davis-Standard in: Paper, Film & Foil Converter 64 (1990) S. 84 - 90 beschrieben. Werkzeuge zum Ausformen der Schmelze zu Folien sind u.a. von Michaeli in: Extrusions-Werkzeuge, Hanser Verlag, München 1991 erläutert.

Was die mindestens eine, auf dem Folienstück angebrachte elektrische Leitung betrifft, so kann diese, vorzugsweise als Leiterbahn, mittels bekannter Druckverfahren oder durch (andere) Dünnschichttechniken für elektronische Schaltungen aufgedruckt sein.

Weiter ist denkbar, dass auch der (mindestens eine) Messfühler, beispielsweise eine Elektrode, durch eine dieser Techniken auf das Folienstück aufgebracht ist. Es ist auch vorstellbar, mittels der Druck- oder Dünnschichttechniken einen oder mehrere eventuell zusätzlich auf dem Folienstück benötigte elektronische (Halbleiter-)Bauelemente, wie etwa Widerstände, Transistoren, Kondensatoren oder dergleichen, aufzudrucken oder mittels der Dünnschichttechniken aufzubringen.

Das Aufdrucken könnte unter anderem durch Inkjet-Drucker oder Siebdrucker mittels an sich bekannter kunststoffbasierter "Tinten" erfolgen, die mit elektrisch leitfähigen Partikeln angereichert sind. Zudem sind Aufbringungsprozesse von leitfähigen "Tinten" oder Materialien mit Membranfunktionen durch einen Dispenserprozess möglich.

Auch ist denkbar, die (mindestens eine) elektrische Leitung und/oder den (mindestens einen) Messfühler und/oder den oder die weiteren elektronischen (Halbleiter-)Bauelemente mittels 3D-Druckern aufzubringen.

Die erfindungsgemäß vorgesehene Verwendung eines Foliensensors für die Sensoreinheit erlaubt einen besonders flachen Aufbau der Sensoreinheit sowie eine kostengünstige Herstellung derselben. Insofern das Sensorteil und das Elektronikteil zudem lösbar miteinander verbunden werden, ist eine dauerhaft besonders hygienische Verwendung der Sensoreinheit möglich. So kann beispielweise vor der Weitergabe der Sensoreinheit an eine weitere Person bzw. vor einer erneuten Verwendung das den Foliensensor umfassende, vergleichsweise kostengünstig herzustellende Sensorteil entfernt und durch ein neues, noch unbenutztes Sensorteil ausgetauscht werden, während das in der Regel aufwendige und kostenintensive Elektronikteil weiterverwendet werden kann. Da in der Regel nur das Sensorteil unmittelbar am Körper anliegt, kann somit Hygieneproblemen auf diese Weise wirksam begegnet werden.

Was die Abmessungen der Sensoreinheit betrifft, so kann die Sensoreinheit bevorzugt eine Länge und/oder eine Breite zwischen 20 mm und 200 mm aufweisen, besonders bevorzugt zwischen 30 mm und 100 mm. Die (Aufbau-)Höhe kann vorzugsweise zwischen 4 mm und 30 mm betragen, besonders bevorzugt zwischen 6 mm und 20 mm. Die (Außen-)Kontur oder (Außen-)Form kann, insbesondere im Grundriss, bevorzugt quadratisch oder rechteckig ausgebildet sein.

Was die Abmessungen des Sensorteils betrifft, so kann das Sensorteil bevorzugt eine Länge und/oder eine Breite zwischen 20 mm und 200 mm aufweisen, besonders bevorzugt zwischen 30 mm und 100 mm. Die (Aufbau-)Höhe kann vorzugsweise zwischen 4 mm und 20 mm betragen, besonders bevorzugt zwischen 6 mm und 15 mm. Die (Außen-)Kontur oder (Außen-)Form kann, insbesondere im Grundriss, bevorzugt rund, quadratisch oder rechteckig ausgebildet sein.

Was die Abmessungen des Elektronikteils betrifft, so kann das Elektronikteil bevorzugt eine Länge und/oder eine Breite zwischen 20 mm und 80 mm aufweisen, besonders bevorzugt zwischen 30 mm und 60 mm. Die (Aufbau-)Höhe kann vorzugsweise zwischen 4 mm und 15 mm betragen, besonders bevorzugt zwischen 6 mm und 12 mm. Die (Außen-)Kontur oder (Außen-)Form kann, insbesondere im Grundriss, bevorzugt rund ausgebildet sein.

Es versteht sich, dass mit der oben genannten (Aufbau-)Höhe der Sensoreinheit die Abmessung derselben gemeint ist, die bei bestimmungsgemäßer Befestigung der Sensoreinheit an der Haut des Körpers die Erstreckung der gesamten Sensoreinheit senkrecht oder zumindest annähernd senkrecht zur Hautoberfläche bestimmen würde.

Mit der (Aufbau-)Höhe des Sensorteils oder des Elektronikteils ist deren jeweilige entsprechende Abmessung in derselben Erstreckungsrichtung wie vorstehend definiert gemeint, allerdings jeweils nur bezogen auf das Sensorteil bzw. das Elektronikteil selbst, also ggf. in einem (möglicherweise hypothetisch) von dem jeweils anderen Bauteil separierten Zustand.

Gemäß einer ersten bevorzugten Weiterbildung der Erfindung weist das Elektronikteil mindestens ein insbesondere elastisches Materialstück bevorzugt aus einem Schaummaterial aus Kunststoff, insbesondere aus Polyurethan, oder einem Elastomermaterial oder einem Silikon auf. Dabei kann die Elektronikeinheit des Elektronikteils teilweise oder vollständig in das Materialstück des Elektronikteils integriert oder eingebettet sein. Dies erlaubt einen guten Schutz des Elektronikteils gegen mögliche schädliche, äußere Einflüsse. Gleichzeitig wird ein solches Material in der Regel von Personen, an deren Haut eine derartige Sensoreinheit befestigt wird - etwa im Vergleich zu einer bekannten Einhausung mittels starrem Hartkunststoff - als angenehm und wenig störend empfunden.

Bei dem Material für das Materialstück kann es sich um jedes Material handeln, das der Fachmann für den oben genannten Zweck auswählen würde, insbesondere zum Abpuffern von Stößen. Bevorzugt ist das elastische Materialstück aus einem wenig bis nicht zytotoxischen Material. Bevorzugt ist das Material ausgewählt aus der Gruppe bestehend aus einem Gel, einem Hydrogel, einem Schaum oder einer Kombination aus beiden.

Bevorzugt ist das zur Einbettung vorgesehene Material ein thermoplastisch verformbarer Schaum. Das Schaummaterial ist bevorzugt ausgewählt aus der Gruppe bestehend aus einem Silikon, einem Polystyrol (PS), einem Polypropylen (PP), einem Polyvinylchlorid (PVC), einem Polyurethan (PU), einem thermoplastischen Polyurethan (TPU), einem Chloropren, einem Naturkautschuk, einem Acrylnitril-Butadien-Kautschuk oder einer Kombination aus mindestens zwei hiervon. Bevorzugt beinhaltet das Schaummaterial ein thermoplastisches Polyurethan.

Bevorzugt ist das Schaummaterial hergestellt durch Mischen einer ersten Polyurethan-Dispersion (A) mit wenigstens einer zweiten Polyurethan-Dispersion (B), gegebenenfalls unter Zusatz weiterer Additive, Aufschäumen und anschließendem Trocknen der Mischung, wobei die Polyurethan-Dispersion (A) erhältlich ist, indem
A) isocyanatfunktionelle Prepolymere aus
   A1) organischen Polyisocyanaten
   A2) polymeren Polyolen mit zahlenmittleren Molekulargewichten von 400 bis 8000 g/mol, vorzugsweise 400 bis 6000 g/mol, oder bevorzugt von 600 bis 3000 g/mol, und OH-Funktionalitäten von 1,5 bis 6, bevorzugt 1,8 bis 3, oder bevorzugt von 1,9 bis 2,1, und
   A3) gegebenenfalls hydroxyfunktionellen Verbindungen mit Molekulargewichten von 62 bis 399 g/mol, und
   A4) gegebenenfalls isocyanatreaktiven, anionischen oder potentiell anionischen und gegebenenfalls nichtionischen Hydrophilierungsmitteln,
   hergestellt, und
B) deren freie NCO-Gruppen dann ganz oder teilweise
   B1) gegebenenfalls mit aminofunktionellen Verbindungen mit Molekulargewichten von 32 bis 400 g/mol und
   B2) mit aminofunktionellen, anionischen oder potentiell anionischen Hydrophilierungsmitteln
unter Kettenverlängerung umgesetzt werden und die Prepolymere vor, während oder nach Schritt B) in Wasser dispergiert werden, wobei gegebenenfalls enthaltene potentiell anionische Gruppen durch teilweise oder vollständige Umsetzung mit einem Neutralisationsmittel in die anionische Form überführt werden, und
wobei die zweite Polyurethan-Dispersion (B) erhältlich ist durch reaktive Umsetzung wenigstens folgender Komponenten:
A. einer aliphatischen Polyisocyanat-Komponente mit einer mittleren Isocyanatfunktionalität von ≥ 1,8 und ≤ 2,6,
B. einer polymeren Polyether-Polyol-Komponente;
C. einer aminofunktionellen Kettenverlängerer-Komponente mit mindestens 2 isocyanatreaktiven Aminogruppen, enthaltend wenigstens eine aminofunktionelle Verbindung C1., die keine ionischen oder ionogenen Gruppen aufweist und/oder eine aminofunktionelle Verbindung C2., die ionische oder ionogene Gruppen aufweist,
D. gegebenenfalls weiteren hydrophilierenden Komponenten, die unterschiedlich sind von C2.,
E. gegebenenfalls hydroxyfunktionellen Verbindungen mit einem Molekulargewicht von 62 bis 399 mol/g,
F. gegebenenfalls weiteren polymeren Polyolen, welche unterschiedlich sind von B.,
G. einer Verbindung, die genau eine isocyanatreaktive Gruppe aufweist, oder einer Verbindung, die mehr als eine isocyanatreaktive Gruppe aufweist, wobei nur eine der isocyanatreaktiven Gruppen unter den gewählten Umsetzungsbedingungen mit den in der Reaktionsmischung vorhandenen Isocyanatgruppen reagiert und
H. optional einer aliphatischen Polyisocyanat-Komponente mit einer mittleren Isocyanatfunktionalität von > 2,6 und ≤ 4,
wobei die Komponenten B. und F. zusammen ≤ 30 Gew.-% an Komponente F., bevorzugt ≤ 20 Gew.-% an Komponente F., oder bevorzugt ≤ 10 Gew.-% an Komponente F., bezogen auf die Gesamtmasse der Komponenten B. und F. enthalten.

Beispiele für die Herstellung, mögliche Zusammensetzungen und Eigenschaften des geschäumten Artikels, hier als Schaummaterial bezeichnet, die aus der reaktiven Umsetzung der ersten Polyurethan-Dispersion (A) hergestellt aus dem Prepolymer A) aus den Komponenten A1) bis A4), sowie B1), B2), mit der zweiten Polyurethan-Dispersion (B) aus den Komponenten A. bis H. entsteht, können der Publikation WO 2020/173760 entnommen werden, insbesondere den Seiten 2 bis 23.

Zusätzlich oder alternativ kann auch das Sensorteil ein solches Materialstück aus einem solchen, wie vorstehend beschriebenen Kunststoff-Schaummaterial oder Elastomermaterial oder Silikon aufweisen. Dabei kann das Folienstück des Foliensensors insbesondere stoffschlüssig mit dem Materialstück des Sensorteils unlösbar bzw. fest verbunden sein, insbesondere mit einer Außenfläche desselben. Die Verbindung kann beispielsweise durch Verklebung erfolgen, besonders bevorzugt mittels eines doppelseitig klebenden Klebematerialstücks beziehungsweise über eine Klebeschicht. Alternativ könnte das Folienstück auch auf das Materialstück auflaminiert sein. Verschiedene Verbindungsvarianten sind hier denkbar.

Was die ggf. lösbare Verbindung von Elektronikteil und Sensorteil betrifft, so kann das Sensorteil mit dem Elektronikteil kraft-, stoff- und/oder formschlüssig verbunden sein. Insbesondere kann es mit diesem lösbar verklebt sein, etwa mittels eines doppelseitig klebenden Klebematerialstücks.

Als Material für eine solche Verklebung von Elektronikteil und Sensorteil kann jedes Material eingesetzt werden, das der Fachmann hierfür auswählen würde. Dieses Material kann bevorzugt einen Klebstoff umfassen oder als solcher ausgebildet sein, der ausgewählt ist aus der Gruppe bestehend aus Acrylklebstoff, ein (thermoplastischer) Polyurethanklebstoff, ein Epoxidharz-Klebstoff, ein Silikonklebstoff, ein Kautschukklebstoff oder eine Mischung aus mindestens zwei hiervon. Bevorzugt wird einer der Polyurethanklebstoffe verwendet wie er in den Publikationen zu EP 3 502 156 A1 oder US 2019/0233691 A1 beschrieben ist. Denkbar ist insbesondere, dass das Material zur Verklebung einen nicht zytotoxischen Klebstoff umfasst oder als solcher ausgebildet ist.

Gemäß einer weiteren bevorzugten Ausführungsform der Erfindung kann das Materialstück des Sensorteils eine Vertiefung aufweisen, in der das Elektronikteil angeordnet ist. Dies insbesondere unter lösbarer Verbindung des Elektronikteils, insbesondere des Materialstücks desselben, mit dem Materialstück des Sensorteils durch ein bzw. das doppelseitig klebende Klebematerialstück. Diese Variante erlaubt einen besonders kompakten Aufbau der Sensoreinheit und eröffnet die Möglichkeit, auf störende Vorsprünge etc. weitgehend verzichten zu können.

Die Vertiefung des Materialstücks des Sensorteils kann dabei durch eine durch das Materialstück gebildete Bodenwand begrenzt sein sowie durch eine mit dieser Bodenwand einstückig verbundene, durch das Materialstück des Sensorteils gebildete, die Vertiefung seitlich umgebende Seitenwand.

Die Vertiefung kann dabei unter Bildung der Bodenwand und der Seitenwand beispielsweise durch Prägung in das Materialstück eingebracht sein.

Vorzugsweise kann das Materialstück des Elektronikteils die Vertiefung des Sensorteils zu einer (ansonsten offenen) Seite der Vertiefung hin verschließen. Insbesondere zu einer Seite, die der zu der Vertiefung zeigenden Seite der Bodenwand gegenüberliegt.

Das Materialstück des Elektronikteils kann kraftschlüssig mit der die Vertiefung seitlich umgebenden Seitenwand verbunden sein. Vorzugsweise, indem das Materialstück des Elektronikteils unter kraftschlüssiger Anlage an die Seitenwand mit (seitlichem) Übermaß in die Vertiefung eingebracht ist.

Die Außenkontur des Materialstücks des Elektronikteils kann im Übrigen vorteilhafterweise an die Innenkontur der die Vertiefung des Materialstücks des Sensorteils seitlich umgebenden Seitenwand angepasst sein. Beispielsweise könnte die Außenkontur des Materialstücks des Elektronikteils nach Art eines Puzzleteils ausgebildet sein oder auch eine sonstige Freiform aufweisen, die sich dann in abgestimmter Form in der Innenkontur der Seitenwand wiederfindet. Während der Herstellung der Sensoreinheit oder wenn das Sensorteil ausgetauscht werden soll, kann dann das Materialstück des Elektronikteils (insbesondere mit Übermaß) in die Vertiefung mit entsprechend konturierter Seitenwand gedrückt bzw. eingebracht werden.

Gemäß einer weiteren Ausführungsform der Erfindung kann vorgesehen sein, dass ein erster Abschnitt des Folienstücks des Foliensensors einer ersten Seite des Materialstücks des Sensorteils zugeordnet (vorzugsweise an dieser angeordnet) und insbesondere mit dieser (bevorzugt unlösbar) verbunden ist, insbesondere der der Vertiefung zugewandten Innenseite der Bodenwand, und dass ein zweiter Abschnitt des Folienstücks des Foliensensors, einer, - bei bestimmungsgemäßer Verwendung der Sensoreinheit - dem Körper zugewandten, zweiten Seite des Materialstücks des Sensorteils zugeordnet (vorzugsweise an dieser angeordnet) und insbesondere mit dieser (bevorzugt unlösbar) verbunden ist, insbesondere der der Innenseite vorzugsweise parallel gegenüberliegenden Außenseite der Bodenwand.

Dabei können die beiden Abschnitte des Folienstücks durch einen Verbindungsabschnitt des Folienstücks insbesondere einstückig miteinander verbunden sein, der unter Ausbildung eines Umschlagbereichs von der ersten Seite zur zweiten Seite des Materialstücks des Sensorteils geführt ist, in dem der Verbindungsabschnitt insbesondere um 180°, bevorzugt u-förmig, um eine Kante eines vorzugsweise plattenförmigen Kantenbereichs des Materialstücks des Sensorteils umgeschlagen ist.

Der Messfühler des Foliensensors kann an einer - bei bestimmungsgemäßer Verwendung der Sensoreinheit - dem Körper zugewandten Außenseite des Sensorteils, insbesondere des Materialstücks des Sensorteils, angeordnet sein. Vorzugsweise kann es an der Außenseite der die Vertiefung begrenzenden Bodenwand des Sensorteils angeordnet sein.

Dabei kann der Messfühler bevorzugt an dem zweiten Abschnitt des Folienstücks des Foliensensors angeordnet sein.

Was die Elektronikeinheit des Elektronikteils betrifft, so kann diese bevorzugt eine insbesondere flexible Leiterplatte mit ein oder mehreren elektronischen Bauteilen aufweisen, insbesondere mit ein oder mehreren Messelektronikbauteilen und/oder mit einer Energieversorgungseinheit, insbesondere einer Batterie, zum Betreiben der Elektronikeinheit und/oder mit einer Sende- und/oder Empfangseinheit zum drahtlosen Übertragen von Informationen an eine entfernte Sende- und/oder Empfangseinheit.

Zur elektrischen Verbindung von Elektronikteil und Sensorteil kann die Elektronikeinheit ein oder mehrere elektrische Kontaktelemente aufweisen, die lösbar elektrisch verbunden sind mit ein oder mehreren zugeordneten elektrischen, insbesondere an dem Folienstück des Foliensensors des Sensorteils angeordneten Kontaktelementen des Foliensensors.

Das oder die elektrischen Kontaktelemente der Elektronikeinheit des Elektronikteils können dabei an einer Seite der Leiterplatte angeordnet sein, die dem Materialstück des Sensorteils zugewandt ist, insbesondere der die Vertiefung des Materialstücks des Sensorteils begrenzenden Bodenwand.

Die an der Leiterplatte angeordneten elektronischen Bauteile der Elektronikeinheit können an einer von dem Materialstück des Sensorteils abgewandten Seite der Leiterplatte angeordnet sein, insbesondere an einer von der Bodenwand des Materialstücks des Sensorteils abgewandten Seite.

Dabei kann zwischen dem Materialstück des Sensorteils und dem Materialstück des Elektronikteils ein flächiges, elektrisch leitfähiges Material oder Materialstück angeordnet sein, wobei das oder die elektrischen Kontaktelemente der Elektronikeinheit an einer ersten Seite dieses flächigen Materials oder Materialstücks anliegen und mit dieser elektrisch leitend verbunden sind, und wobei das oder die Kontaktelemente des Foliensensors an einer zweiten, der ersten Seite gegenüberliegenden Seite des leitfähigen Materials oder Materialstücks anliegen und mit dieser elektrisch leitend verbunden sind. Dementsprechend stellt das elektrische leitfähige Material oder Materialstück in dieser Variante eine elektrische Verbindung zwischen den Kontaktelementen der Elektronikeinheit und den Kontaktelementen des Foliensensors her.

Das flächige, elektrisch leitfähige Materialstück kann beispielsweise ein oder das doppelseitig klebende Klebematerialstück sein.

Was den Foliensensor betrifft, so kann dieser, insbesondere der der zweiten Seite des Materialstücks des Sensorteils zugeordnete zweite Abschnitt des Folienstücks des Foliensensors, ein oder mehrere Messfühler aufweisen, nämlich mindestens einen Temperatursensor und/oder einen Leitfähigkeitssensor und/oder einen Drucksensor und/oder einen Impedanzsensor. Mit ein oder mehreren dieser oder anderer Sensoren können dann Messwerte erfasst werden, auf Basis derer die zu erfassenden Körperparameter bestimmt werden.

Gemäß einer weiteren Ausführungsform der Erfindung kann vorgesehen sein, dass das Materialstück des Sensorteils eine sich insbesondere von seiner ersten Seite bis zu seiner zweiten Seite erstreckende Durchtrittsöffnung aufweist, durch die sich die Hauptblickrichtung einer insbesondere dem Elektronikteil zugeordneten, vorzugsweise auf der Leiterplatte der Elektronikeinheit des Elektronikteils angeordneten, elektrooptischen, vorzugsweise eine elektronische Kamera umfassende Erfassungseinrichtung erstreckt.

Dabei kann die Durchtrittsöffnung zum Schutz der Kamera durch eine insbesondere an der ersten und/oder an der zweiten Seite des Materialstücks des Sensorteils angeordnete, transparente Schutzschicht überdeckt sein.

Die zweite Seite des Materialstücks des Sensorteils kann des Weiteren ein oder mehrere, gegenüber mindestens einem anderen, insbesondere ebenen Bereich dieser zweiten Seite hervorstehende Erhebungen aufweisen, wobei der oder jeder Messfühler auf einer solchen Erhebung angeordnet ist. Diese Erhebungen können bei Befestigung der Sensoreinheit an dem Körper zu einem hohen Anpressdruck der Messfühler an dem Körper und entsprechend zu einer guten, die nachfolgenden Messungen optimierenden Verbindung zwischen Messfühler und Körper führen.

Weitere Merkmale der vorliegenden Erfindung ergeben sich aus den beigefügten Patentansprüchen, der nachfolgenden Beschreibung bevorzugter Ausführungsbeispiele sowie aus den beigefügten Zeichnungen. Darin zeigt:
- Fig. 1a: eine Schnittdarstellung einer ersten Ausführungsform einer erfindungsgemäßen Sensoreinheit aus Sensorteil und Elektronikteil, in (noch) unverbundenem Zustand der die Sensoreinheit bildenden Einzelteile,
- Fig. 1b: die Schnittdarstellung entsprechend Fig. 1a, in verbundenem bzw. zusammengesetztem Zustand der die Sensoreinheit bildenden Einzelteile,
- Fig. 2a: eine Schnittdarstellung einer zweiten Ausführungsform einer erfindungsgemäßen Sensoreinheit aus Sensorteil und Elektronikteil, in unverbundenem Zustand der die Sensoreinheit bildenden Einzelteile,
- Fig. 2b: die Schnittdarstellung entsprechend Fig. 2a, in verbundenem Zustand der die Sensoreinheit bildenden Einzelteile,
- Fig. 3a: eine Schnittdarstellung einer dritten Ausführungsform einer erfindungsgemäßen Sensoreinheit aus Sensorteil und Elektronikteil, in unverbundenem Zustand der die Sensoreinheit bildenden Einzelteile,
- Fig. 3b: die Schnittdarstellung entsprechend Fig. 3a, in weitgehend verbundenem Zustand der die Sensoreinheit bildenden Einzelteile,
- Fig. 4a: eine Schnittdarstellung einer vierten Ausführungsform einer erfindungsgemäßen Sensoreinheit aus Sensorteil und Elektronikteil, in unverbundenem Zustand der die Sensoreinheit bildenden Einzelteile,
- Fig. 4b: die Schnittdarstellung entsprechend Fig. 4a, in verbundenem Zustand der die Sensoreinheit bildenden Einzelteile,
- Fig. 5a: eine Schnittdarstellung einer fünften Ausführungsform einer erfindungsgemäßen Sensoreinheit aus Sensorteil und Elektronikteil, in unverbundenem Zustand der die Sensoreinheit bildenden Einzelteile,
- Fig. 5b: die Schnittdarstellung entsprechend Fig. 5a, in verbundenem Zustand der die Sensoreinheit bildenden Einzelteile,
- Fig. 6: eine Explosionszeichnung der in den Fig. 5a und 5b gezeigten fünften Ausführungsform.

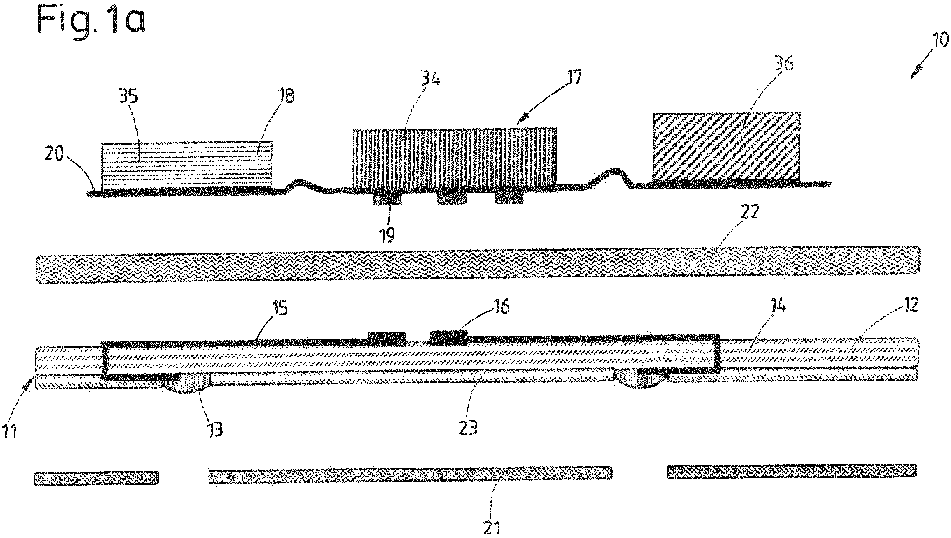

Die in den Fig. 1a-5b gezeigte Sensoreinheit 10 ist dazu vorgesehen und ausgebildet, an einem menschlichen (oder tierischen) Körper befestigt zu werden, um Körperparameter zu erfassen. Bei solchen Körperparametern kann es sich beispielsweise um elektrische Aktivitäten des Herzens oder des Gehirns handeln. Auch die Erfassung diverser anderer Körperparameter ist naturgemäß denkbar.

Nachfolgend werden zunächst die Bauteile einer solchen Sensoreinheit 10 beschrieben, die sämtliche der in Fig. 1a-5b gezeigten Ausführungsformen derselben aufweisen. Anschließend wird auf die Unterschiede der einzelnen Ausführungsformen eingegangen.

Die Sensoreinheit 10 jeder Ausführungsform umfasst ein Sensorteil 11 mit einem Foliensensor 12 mit vorliegend mehreren Messfühlern 13 (es versteht sich, dass auch ein Messfühler 13 genügen kann) sowie ein mit dem Sensorteil 11 vorliegend lösbar verbundenes Elektronikteil 17 mit einer Elektronikeinheit 18 zum Betreiben des Foliensensors 12.

Die Messfühler 13 sind dabei an einem für diese als Träger dienenden, flexiblen Folienstück 14 des Foliensensors 12 des Sensorteils 11 angeordnet bzw. mit diesem Folienstück 14 fest verbunden, und zwar auf einer von dem Elektronikteil 17 abgewandten Seite des Folienstücks 14.

Weiter sind die Messfühler 13 mittels auf dem Folienstück 14 angeordneter elektrischer Leitungen 15 mit ebenfalls auf dem Folienstück 14 befindlichen, zur Kontaktierung des Foliensensors 12 dienenden (elektrischen) Kontaktelementen 16 elektrisch leitend verbunden.

Die Messfühler 13, die elektrischen Leitungen 15 und/oder die Kontaktelemente 16 können beispielsweise mittels additiver Drucktechnik auf das Folienstück 14 aufgebracht sein.

Es versteht sich im Übrigen, dass es auch im Rahmen der vorliegenden Erfindung liegt, die Messfühler 13 und/oder die Kontaktelemente 16 außerhalb des Folienstücks 14 anzuordnen, beispielsweise unmittelbar benachbart zu diesem.

In der Regel wird die Sensoreinheit 10 bzw. das Sensorteil 11 der Sensoreinheit 10 derart an der Haut der Person, deren Körperparameter erfasst werden soll, befestigt, dass die Messfühler 13 unmittelbar an der Haut anliegen.

Wie (nur) anhand der Ausführungsformen der Fig. 1a-3b explizit gezeigt, können dabei zur Befestigung der Sensoreinheit 10 an dem jeweiligen Körper beispielsweise flächige, hautverträgliche, doppelseitig klebende Klebematerialstücke 21 (doppelseitiges Klebeband) verwendet werden, die mit ihrer einen Seite entsprechend mit der dem Körper zugewandten Seite des Sensorteils 11 klebend verbunden werden und mit ihrer anderen Seite mit der Haut des jeweiligen Körpers.

Zur elektrischen Isolierung ist des Weiteren auf der von dem Elektronikteil 17 abgewandten Seite des Folienstücks 14 bzw. des Foliensensors 12 des Sensorteils 11, nämlich auf der bei bestimmungsgemäßer Anordnung dem Körper zugewandten Seite, eine Isolationsschicht 23 vorgesehen, die insbesondere die elektrischen Leitungen 15 nach außen bzw. zum Körper hin überdeckt und elektrisch isoliert.

Was die Elektronikeinheit 18 des Elektronikteils 17 betrifft, so verfügt diese über eine auf einer im vorliegenden Fall flexiblen Leiterplatte 20 angeordnete Messelektronikeinheit 34, an die die Messfühler 13 des Sensorteils 11 elektrisch angeschlossen sind und die die (Mess-)Signale der Messfühler 13 empfängt und ggf. auswertet.

Auf der Leiterplatte 20 ist des Weiteren eine vorliegend als Funkmodul ausgebildete Sendeeinheit 35 angeordnet zum drahtlosen Senden von Signalen der Messfühler 13 bzw. von auf diesen basierenden Informationen an eine entsprechende (nicht dargestellte) entfernte Empfangseinheit.

Weiter umfasst die Elektronikeinheit 18 eine vorliegend als Batterie ausgebildete Energie- bzw. Spannungsversorgung 36 für die einzelnen Bauteile der Elektronikeinheit 18 bzw. des Foliensensors 12.

Die vorgenannten Bauteile sind jeweils auf einer ersten, von dem Sensorteil 11 abgewandten Seite der Leiterplatte 20 angeordnet bzw. befestigt.

Auf der gegenüberliegenden, dem Sensorteil 11 zugewandten Seite der Leitplatte 20 verfügt diese über elektrische Kontaktelemente 19, die mit den elektrischen Kontaktelementen 16 des Foliensensors 12 elektrisch leitend verbunden sind.

Zur lösbaren Befestigung des Elektronikteils 17 an dem Sensorteil 11 ist - ähnlich wie zur Befestigung der Sensoreinheit 10 an dem Körper der entsprechenden Person - vorliegend (mindestens) ein doppelseitiges Klebematerialstück 22 vorgesehen, das unter Anlage (auch) an die Kontaktelemente 19 einerseits mit der dem Sensorteil 11 zugewandten Seite der Leiterplatte 20 verbunden bzw. verklebt ist, andererseits (auch) unter Anlage an die Kontaktelemente 16 mit der der Leiterplatte 20 bzw. dem Elektronikteil 17 zugewandten Seite des Folienstücks 14 des Foliensensors 12.

Der Klebstoff des doppelseitigen Klebematerialstücks 22 ist derart ausgewählt, dass das Elektronikteil 17 bei Bedarf von dem Sensorteil 11 lösbar ist. Aber auch eine unlösbare Verbindung dieser Bauteile liegt im Rahmen der vorliegenden Erfindung.

Das doppelseitige Klebematerialstück 22 ist elektrisch leitend ausgebildet, sodass über dieses die elektrisch leitende Verbindung zwischen den Kontaktelementen 16 des Foliensensors 12 und den Kontaktelementen 19 der Elektronikeinheit 18 bewirkt wird.

Die Sensoreinheit 10 kann erfindungsgemäß durch einen bzw. den flachen Aufbau des Elektronikteils 17 sowie des Sensorteils 11 insgesamt ebenfalls sehr flach aufgebaut sein bzw. eine geringe (Aufbau-)Höhe aufweisen. In den Fig. entspricht die Höhe der Sensoreinheit 10 derjenigen Abmessung, die die Erstreckung der Sensoreinheit 10 von unten nach oben bestimmt bzw. vorliegend der Distanz zwischen der an dem Körper angeordneten Seite der Sensoreinheit 10 bzw. des Sensorteils 11 und der von dem Körper abgewandten Seite der Sensoreinheit 10 bzw. des Elektronikteils 17.

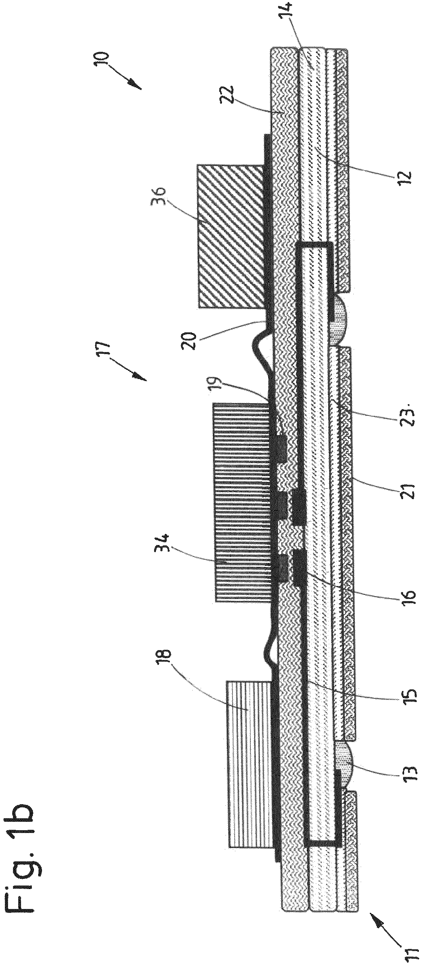

Die Ausführungsform der Fig. 1a, 1b weist über die vorstehend beschriebenen Bauteile keine weiteren Bauteile und/oder Besonderheiten auf.

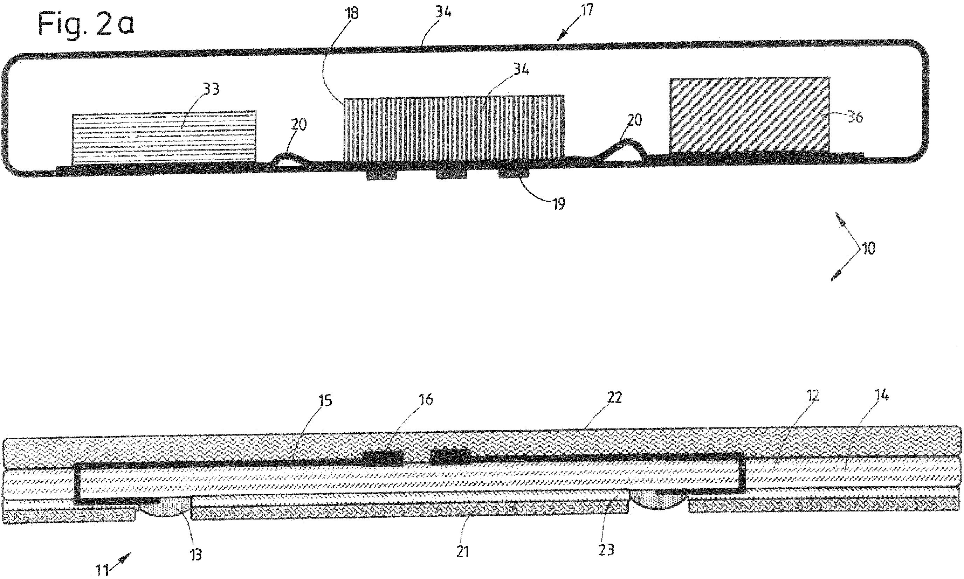

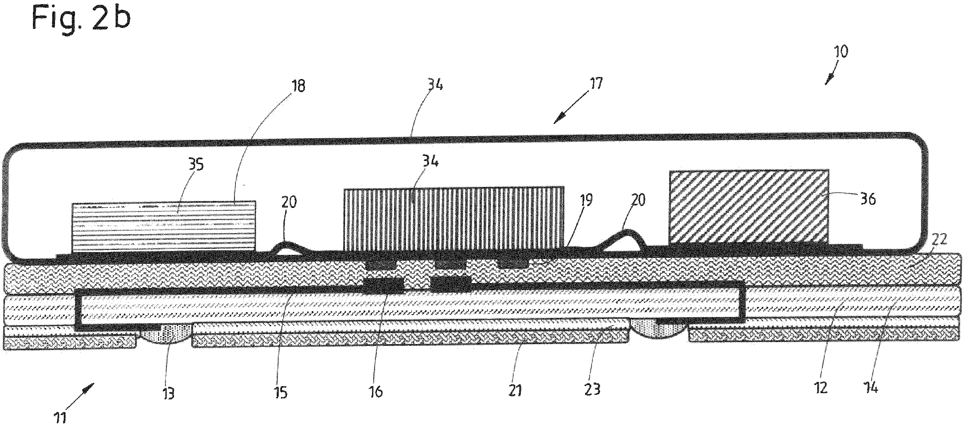

Bei der Ausführungsform der Fig. 2a, 2b dagegen weist das Elektronikteil 17 zusätzlich eine Schutzumhüllung 24 auf, in die die Elektronikeinheit 18 integriert ist und die die Bauteile der Elektronikeinheit ganz oder teilweise schützend umgibt.

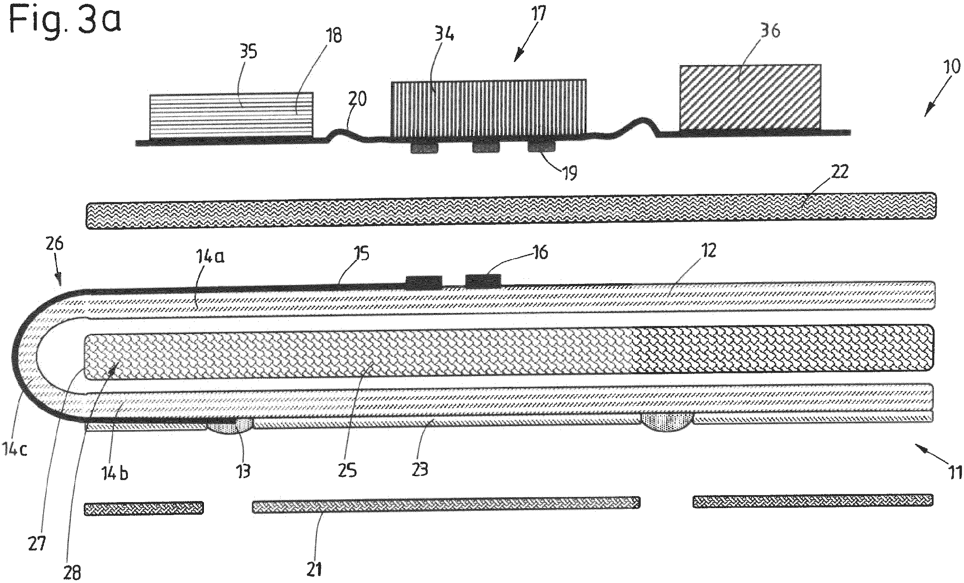

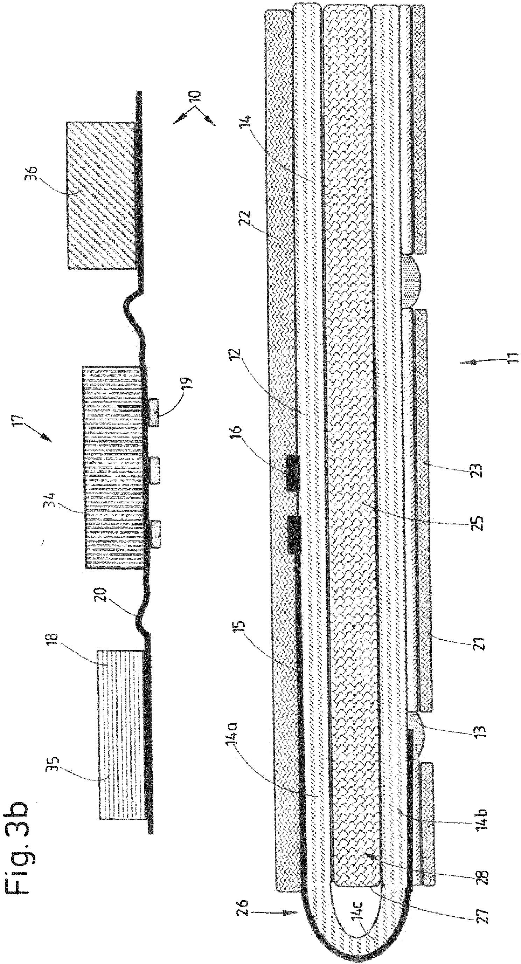

Die Ausführungsform der Fig. 3a, 3b unterscheidet sich von der Ausführungsform der Fig. 1a, 1b dadurch, dass das Sensorteil 11 zusätzlich ein Materialstück 25 aus Kunststoffschaum, etwa PU-Schaum, aufweist, mit dem der Foliensensor 12 vorliegend stoffschlüssig verbunden, beispielsweise verklebt ist. Es versteht sich, dass sich die Erfindung auch auf diverse andere Verbindungsarten zwischen Foliensensor 12 und Sensorteil 11 erstreckt.

Wie erkennbar ist, ist ein erster Abschnitt 14a des Folienstücks 14 des Foliensensors 12 einer dem Elektronikteil 17 zugewandten, ersten Seite des Materialstücks 25 des Sensorteils 11 zugeordnet bzw. an dieser angeordnet und mit dieser Seite fest verbunden.

Ein zweiter Abschnitt 14b des Folienstücks 14 des Foliensensors 12 ist einer bzw. der gegenüberliegenden, dem Körper zugewandten zweiten Seite des Materialstücks 25 des Sensorteils 11 zugeordnet bzw. an dieser angeordnet und mit dieser fest verbunden.

Die beiden Abschnitte 14a, 14b des Folienstücks 14 sind durch einen Verbindungsabschnitt 14c des Folienstücks 14 einstückig miteinander verbunden, und zwar unter Ausbildung eines Umschlagbereichs 26, in dem der Verbindungsabschnitt 14c vorliegend um 180°, hier etwa u-förmig, um eine Kante 27 eines hier plattenförmigen Kantenbereichs 28 des Materialstücks 25 des Sensorteils 11 umgeschlagen ist und auf diese Weise von der ersten Seite zur zweiten Seite des Materialstücks 25 des Sensorteils 11 geführt ist.

Alternativ ist naturgemäß auch denkbar, den Verbindungsabschnitt 14c durch eine geeignete Durchgangsöffnung in dem Materialstück 25 von der ersten zur zweiten Seite zu führen.

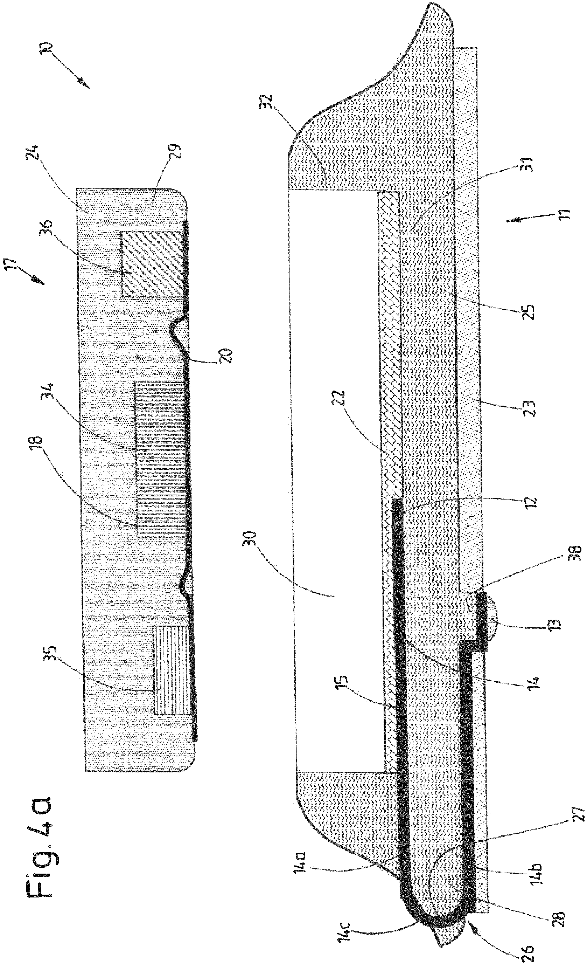

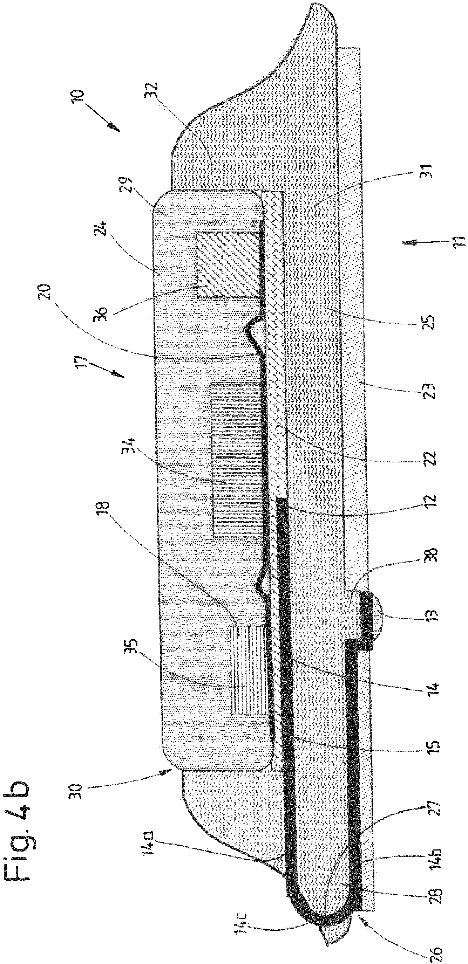

In der Ausführungsform der Fig. 4a, 4b ist die Schutzumhüllung 24 des Elektronikteils 17 - ähnlich wie das Materialstück 25 des Sensorteils 11 - aus einem Materialstück 29 aus Schaummaterial aus Kunststoff, etwa PU-Schaum, gebildet oder auch aus einem Silikon oder einem Elastomermaterial. Die Elektronikeinheit 18 ist dabei ganz oder teilweise in das Materialstück 29 eingebettet.

In das Materialstück 25 des Sensorteils 11 ist zudem eine Vertiefung 30 eingebracht, beispielsweise durch ein Prägeverfahren, in der das Elektronikteil 17 sitzt und dort durch das doppelseitige Klebematerialstück 22 gehalten bzw. befestigt ist.

Die Vertiefung 29 des Materialstücks 25 des Sensorteils 11 ist (nach unten hin) durch eine durch das Materialstück 25 gebildete Bodenwand 31 begrenzt sowie (zur Seite hin) durch eine mit dieser Bodenwand 31 einstückig verbundene, ebenfalls durch das Materialstück 25 gebildete, die Vertiefung 30 seitlich umgebende Seitenwand 32.

Wie erkennbar ist, verschließt das Elektronikteil 17 bzw. das Materialstück 29 die Vertiefung 30 des Sensorteils 11 zu einer (ansonsten offenen) Seite hin, nämlich zu der (oberen) Seite, die der zu der Vertiefung 30 zeigenden Seite der Bodenwand 31 gegenüberliegt.

Das Materialstück 29 des Elektronikteils 17 und die Vertiefung 30 sind bevorzugt derart aufeinander abgestimmt - dies ist aber nicht zwingend erforderlich -, dass das Materialstück 29 kraftschlüssig mit der Seitenwand 32 der Vertiefung 30 verbunden ist. Beispielsweise, indem das Materialstück 29 des Elektronikteils 17 unter kraftschlüssiger Anlage an die Seitenwand 32 mit seitlichem Übermaß in die Vertiefung 30 eingebracht ist.

Erkennbar ist in den Fig. 4a und 4b des Weiteren, dass die von der Vertiefung 30 abgewandte Außenseite der Bodenwand 31 eine gegenüber dem angrenzenden, ebenen Bereich der Außenseite hervorstehende Erhebung 38 aufweist, auf der ein/der Messfühler 13 angeordnet ist, um bei Anordnung der Sensoreinheit 10 an dem jeweiligen Körper die Anlage des Messfühlers 13 an dem Körper zu verbessern.

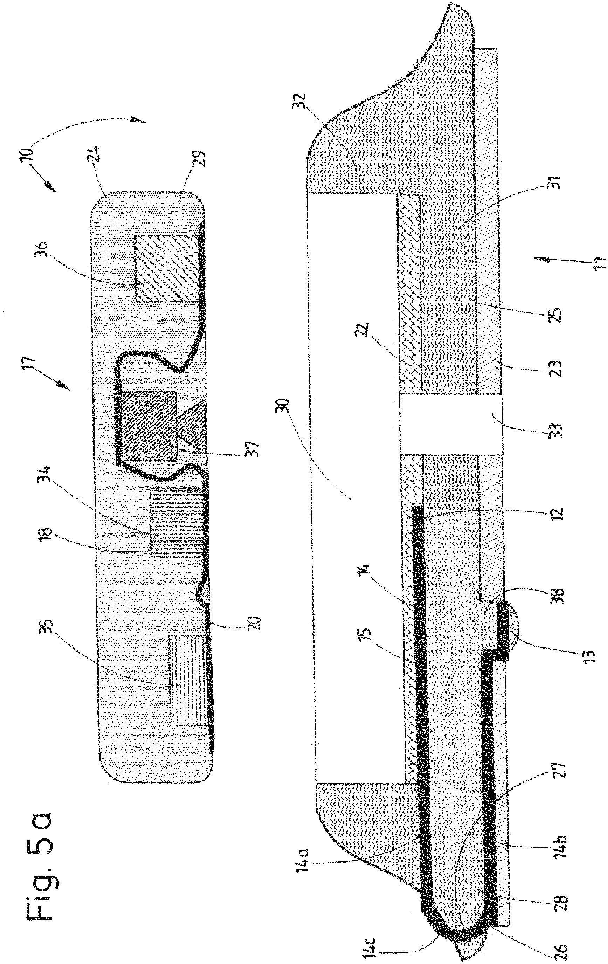

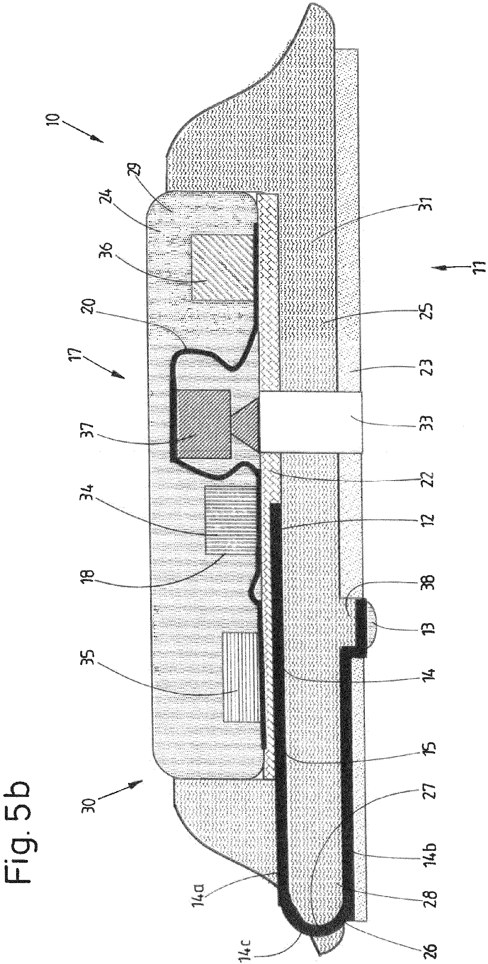

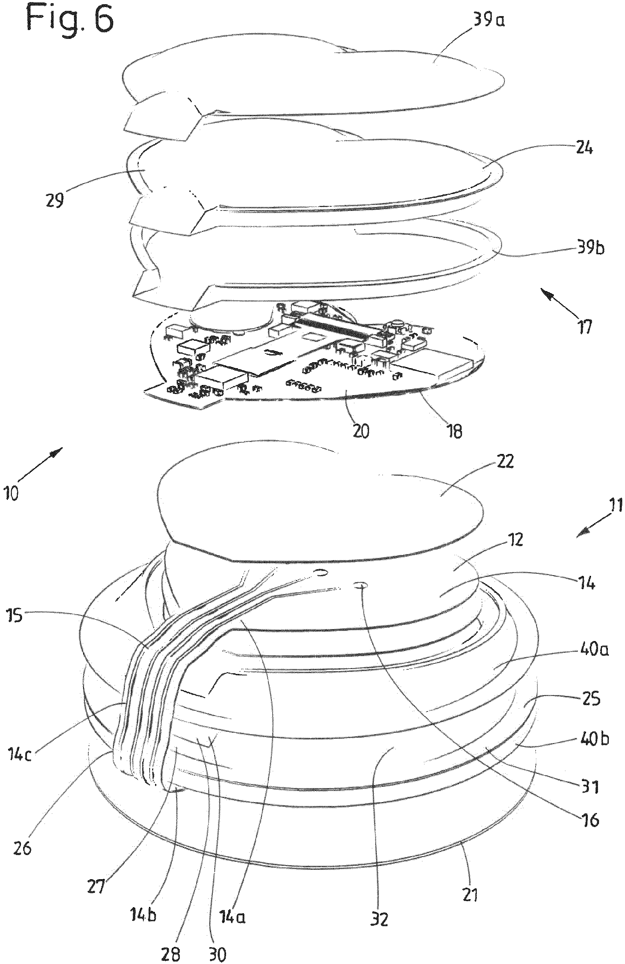

Im Vergleich zu der Ausführungsform der Fig. 4a, 4b verfügt schließlich die Ausführungsform der Fig. 5a, 5b, 6 über eine dem Elektronikteil 17 zugeordnete, elektronische Kamera 37. Konkret ist diese vorliegend auf der Leiterplatte 20 der Elektronikeinheit 18 platziert. Mit dieser Kamera 37 kann beispielsweise die Haut des Körpers der jeweiligen Person optisch erfasst werden.

Sowohl das Elektronikteil 17 als auch das Sensorteil 11 weisen dabei jeweils eine Öffnung auf, die zusammen eine Durchtrittsöffnung 33 bilden, durch die die Hauptblickrichtung der Kamera 33 gerichtet ist und die sich bei bestimmungsgemäßer Verwendung der Sensoreinheit 11 bis zur am Körper angeordneten Außenseite des Sensorteils 11 erstreckt.

Bevorzugt kann diese Durchtrittsöffnung 33 noch durch eine insbesondere an der ersten und/oder an der zweiten Seite des Materialstücks 25 des Sensorteils 11 angeordnete, transparente Schutzschicht bzw. ein entsprechendes transparentes Schutzteil überdeckt sein.

Nur in der Fig. 6 ist erkennbar, dass das Materialstück 29 des Elektronikteils 17 auf zwei gegenüberliegenden Seiten, nämlich auf der von dem Sensorteil 11 abgewandten Seite sowie auf der dem Sensorteil 11 zugewandten Seite, jeweils mit einem (dünnen) Film 39a bzw. 39b versehen ist. Hierbei kann es sich beispielsweise um einen TPU-Film handeln.

In gleicher Weise ist auch das Materialstück 25 des Sensorteils 11 mit einem solchen Film 40a bzw. 40b versehen, nämlich die erste und die zweite Seite des Materialstücks 25, ebenfalls insbesondere mit einem TPU-Film.

Bevorzugte Ausführungsbeispiele:
1. Sensoreinheit zur Befestigung an einem menschlichen oder tierischen Körper, insbesondere an der Haut des Körpers, mit einem Sensorteil (11), das einen Foliensensor (12) zur Messung ein oder mehrerer Körperparameter aufweist, der über ein Folienstück (14) mit mindestens einer an dem Folienstück (14) angeordneten elektrischen Leitung verfügt sowie über einen mit dieser Leitung elektrisch leitend verbundenen, bevorzugt an dem Folienstück(14) angeordneten Messfühler (13), und mit einem mit dem Sensorteil (11) bevorzugt lösbar verbundenen Elektronikteil (17), das eine Elektronikeinheit zum Betreiben des Foliensensors aufweist.
2. Sensoreinheit gemäß Ausführungsbeispiel 1, **dadurch gekennzeichnet,** dass das Elektronikteil (17) und/oder das Sensorteil (11) (jeweils) mindestens ein insbesondere elastisches Materialstück (25, 29) bevorzugt aus einem Schaummaterial aus Kunststoff, insbesondere aus Polyurethan, oder einem Elastomermaterial aufweist.
3. Sensoreinheit gemäß Ausführungsbeispiel 2, **dadurch gekennzeichnet,** dass die Elektronikeinheit teilweise oder vollständig in das Materialstück (29) des Elektronikteils (17) integriert oder eingebettet ist.
4. Sensoreinheit gemäß Ausführungsbeispiel 2 oder 3, **dadurch gekennzeichnet,** dass das Folienstück (14) des Foliensensors (12) insbesondere stoffschlüssig mit dem Materialstück (25) des Sensorteils (11) unlösbar bzw. fest verbunden ist, insbesondere mit einer Außenfläche desselben, bevorzugt durch Verklebung, besonders bevorzugt mittels eines doppelseitig klebenden Klebematerialstücks.
5. Sensoreinheit gemäß einem oder mehreren der vorhergehenden Ausführungsbeispiele, **dadurch gekennzeichnet,** dass das Sensorteil (11) mit dem Elektronikteil (17) kraft-, stoff- und/oder formschlüssig lösbar verbunden ist, insbesondere mit diesem lösbar verklebt ist, vorzugsweise mittels eines doppelseitig klebenden Klebematerialstücks.
6. Sensoreinheit gemäß einem oder mehreren der vorhergehenden Ausführungsbeispiele, mindestens gemäß Ausführungsbeispiel 2, **dadurch gekennzeichnet,** dass das Materialstück (25) des Sensorteils (11) eine Vertiefung (30) aufweist, und dass das Elektronikteil (17) in der Vertiefung (30) angeordnet ist, insbesondere unter lösbarer Verbindung des Elektronikteils (17), insbesondere des Materialstücks (29) desselben, mit dem Materialstück (25) des Sensorteils (11) vorzugsweise durch das doppelseitig klebende Klebematerialstück.
7. Sensoreinheit gemäß einem oder mehreren der vorhergehenden Ausführungsbeispiele, mindestens gemäß Ausführungsbeispiel 2 und 6, **dadurch gekennzeichnet,** dass die Vertiefung (30) des Materialstücks (25) des Sensorteils (11) durch eine durch das Materialstück (25) gebildete Bodenwand begrenzt ist sowie durch eine mit dieser Bodenwand einstückig verbundene, durch das Materialstück (25) des Sensorteils (11) gebildete, die Vertiefung (30) seitlich umgebende Seitenwand.
8. Sensoreinheit gemäß einem oder mehreren der vorhergehenden Ausführungsbeispiele, mindestens gemäß Ausführungsbeispiel 2 oder Ausführungsbeispiel 6, **dadurch gekennzeichnet,** dass das Materialstück (29) des Elektronikteils (17) die Vertiefung (30) des Sensorteils (11) zu einer (ansonsten offenen) Seite hin verschließt, insbesondere zu einer Seite, die der zu der Vertiefung (30) zeigenden Seite der Bodenwand gegenüberliegt.
9. Sensoreinheit gemäß einem oder mehreren der vorhergehenden Ausführungsbeispiele, mindestens gemäß Ausführungsbeispiel 2 oder 7, **dadurch gekennzeichnet,** dass das Materialstück (29) des Elektronikteils (17) kraftschlüssig mit der die Vertiefung (30) seitlich umgebenden Seitenwand verbunden ist, vorzugsweise, indem das Materialstück (29) des Elektronikteils (17) unter kraftschlüssiger Anlage an die Seitenwand mit (seitlichem) Übermaß in die Vertiefung (30) eingebracht ist.
10. Sensoreinheit gemäß einem oder mehreren der vorhergehenden Ausführungsbeispiele, mindestens gemäß Ausführungsbeispiel 2 und 7, **dadurch gekennzeichnet,** dass die Außenkontur des Materialstücks (29) des Elektronikteils (17) an die Innenkontur der die Vertiefung (30) des Materialstücks (25) des Sensorteils (11) seitlich umgebenden Seitenwand angepasst ist.
11. Sensoreinheit gemäß einem oder mehreren der vorhergehenden Ausführungsbeispiele, **dadurch gekennzeichnet,** dass ein erster Abschnitt des Folienstücks (14) des Foliensensors (12) einer ersten Seite des Materialstücks (25) des Sensorteils (11) zugeordnet ist und insbesondere mit dieser (bevorzugt unlösbar) verbunden ist, insbesondere der der Vertiefung (30) zugewandten Innenseite der Bodenwand, und dass ein zweiter Abschnitt des Folienstücks (14) des Foliensensors (12), einer, - bei bestimmungsgemäßer Verwendung der Sensoreinheit - dem Körper zugewandten, zweiten Seite des Materialstücks (25) des Sensorteils (11) zugeordnet und insbesondere mit dieser (bevorzugt unlösbar) verbunden ist, insbesondere der der Innenseite vorzugsweise parallel gegenüberliegenden Außenseite der Bodenwand.
12. Sensoreinheit gemäß Ausführungsbeispiel 11, **dadurch gekennzeichnet,** dass die beiden Abschnitte des Folienstücks (14) durch einen Verbindungsabschnitt des Folienstücks (14) insbesondere einstückig miteinander verbunden sind, der unter Ausbildung eines Umschlagbereichs, in dem der Verbindungsabschnitt insbesondere um 180°, bevorzugt u-förmig, um eine Kante eines vorzugsweise plattenförmigen Kantenbereichs des Materialstücks (25) des Sensorteils (11) umgeschlagen ist, von der ersten Seite zur zweiten Seite des Materialstücks (25) des Sensorteils (11) geführt ist.
13. Sensoreinheit gemäß einem oder mehreren der vorhergehenden Ausführungsbeispiele, **dadurch gekennzeichnet,** dass der Messfühler (13) des Foliensensors (12) an einer - bei bestimmungsgemäßer Verwendung der Sensoreinheit - dem Körper zugewandten Außenseite des Sensorteils (11) angeordnet ist, insbesondere des Materialstücks (25) des Sensorteils (11), vorzugsweise an der Außenseite der die Vertiefung (30) begrenzenden Bodenwand des Sensorteils (11).
14. Sensoreinheit gemäß einem oder mehreren der vorhergehenden Ausführungsbeispiele, mindestens gemäß Ausführungsbeispiel 11, **dadurch gekennzeichnet,** dass der Messfühler (13) an dem zweiten Abschnitt des Folienstücks (14) des Foliensensors (12) angeordnet ist.
15. Sensoreinheit gemäß einem oder mehreren der vorhergehenden Ausführungsbeispiele, **dadurch gekennzeichnet,** dass die Elektronikeinheit des Elektronikteils (17) eine insbesondere flexible Leiterplatte (20) mit ein oder mehreren elektronischen Bauteilen aufweist, insbesondere mit ein oder mehreren Messelektronikbauteilen und/oder einer Energieversorgungseinheit, insbesondere einer Batterie, zum Betreiben der Elektronikeinheit (17) und/oder einer Sende- und/oder Empfangseinheit zum drahtlosen Übertragen von Informationen an eine entfernte Sende- und/oder Empfangseinheit.
16. Sensoreinheit gemäß einem oder mehreren der vorhergehenden Ausführungsbeispiele, **dadurch gekennzeichnet,** dass zur elektrischen Verbindung von Elektronikteil (17) und Sensorteil (11) die Elektronikeinheit ein oder mehrere elektrische Kontaktelemente aufweist, die lösbar elektrisch verbunden sind mit ein oder mehreren zugeordneten elektrischen, insbesondere an dem Folienstück (14) des Foliensensors (12) des Sensorteils (11) angeordneten Kontaktelementen des Foliensensors (12).
17. Sensoreinheit gemäß Ausführungsbeispiel 15 oder 16, **dadurch gekennzeichnet,** dass das oder die elektrischen Kontaktelemente der Elektronikeinheit des Elektronikteils (17) an einer Seite der Leiterplatte (20) angeordnet sind, die dem Materialstück (25) des Sensorteils (11) zugewandt ist, insbesondere der die Vertiefung (30) des Materialstücks (25) des Sensorteils (11) begrenzenden Bodenwand.
18. Sensoreinheit gemäß einem oder mehreren der vorhergehenden Ausführungsbeispiele, zumindest gemäß Ausführungsbeispiel 15, **dadurch gekennzeichnet,** dass die an der Leiterplatte (20) angeordneten elektronischen Bauteile der Elektronikeinheit an einer von dem Materialstück (25) des Sensorteils (11) abgewandten Seite der Leiterplatte (20) angeordnet sind, insbesondere an einer von der Bodenwand des Materialstücks (25) des Sensorteils (11) abgewandten Seite.
19. Sensoreinheit gemäß einem oder mehreren der vorhergehenden Ausführungsbeispiele, mindestens gemäß Ausführungsbeispiel 16, **dadurch gekennzeichnet,** dass zwischen dem Materialstück (25) des Sensorteils (11) und dem Materialstück (29) des Elektronikteils (17) ein flächiges, elektrisch leitfähiges Material oder Materialstück angeordnet ist, wobei das oder die elektrischen Kontaktelemente der Elektronikeinheit an einer ersten Seite dieses flächigen Materials oder Materialstücks anliegen und mit dieser elektrisch leitend verbunden sind, und wobei das oder die Kontaktelemente des Foliensensors (12) an einer zweiten, der ersten Seite gegenüberliegenden Seite des leitfähigen Materials oder Materialstücks anliegen und mit dieser elektrisch leitend verbunden sind.
20. Sensoreinheit gemäß Ausführungsbeispiel 19, **dadurch gekennzeichnet,** dass das flächige, elektrisch leitfähige Materialstück (25) ein oder das doppelseitig klebende Klebematerialstück ist.
21. Sensoreinheit gemäß einem oder mehreren der vorhergehenden Ausführungsbeispiele, mindestens gemäß Ausführungsbeispiel 2 oder 7, **dadurch gekennzeichnet,** dass die Vertiefung (30) des Materialstücks (25) des Sensorteils (11) unter Bildung der Bodenwand und der Seitenwand durch Prägung in das Materialstück eingebracht ist.
22. Sensoreinheit gemäß einem oder mehreren der vorhergehenden Ausführungsbeispiele, **dadurch gekennzeichnet,** dass der Foliensensor (14), insbesondere der der zweiten Seite des Materialstücks (25) des Sensorteils (11) zugeordnete zweite Abschnitt des Folienstücks des Foliensensors (12), ein oder mehrere Messfühler (13) aufweist, nämlich mindestens einen Temperatursensor und/oder einen Leitfähigkeitssensor und/oder einen Drucksensor und/oder einen Impedanzsensor.
23. Sensoreinheit gemäß einem oder mehreren der vorhergehenden Ausführungsbeispiele, **dadurch gekennzeichnet,** dass das Materialstück (25) des Sensorteils (11) eine sich insbesondere von seiner ersten Seite bis zu seiner zweiten Seite erstreckende Durchtrittsöffnung aufweist, durch die sich die Hauptblickrichtung einer insbesondere dem Elektronikteil (17) zugeordneten, vorzugsweise auf der Leiterplatte (20) der Elektronikeinheit des Elektronikteils (17) angeordneten, vorzugsweise eine elektronische Kamera umfassenden, elektrooptischen Erfassungseinrichtung erstreckt.
24. Sensoreinheit gemäß Ausführungsbeispiel 23, **dadurch gekennzeichnet,** dass die Durchtrittsöffnung durch eine insbesondere an der ersten und/oder an der zweiten Seite des Materialstücks (25) des Sensorteils (11) angeordnete, transparente Schutzschicht überdeckt ist.
25. Sensoreinheit gemäß einem oder mehreren der vorhergehenden Ausführungsbeispiele, **dadurch gekennzeichnet,** dass die zweite Seite des Materialstücks (25) des Sensorteils (11) ein oder mehrere, gegenüber mindestens einem anderen, insbesondere ebenen Bereich dieser zweiten Seite hervorstehende Erhebungen (38) aufweist, und dass der oder jeder Messfühler (13) auf einer solchen Erhebung (38) angeordnet ist.
26. Sensorteil für eine Sensoreinheit gemäß einem oder mehreren der vorhergehenden Ausführungsbeispiele, das einen Foliensensor (12) zur Messung mindestens eines Körperparameters aufweist, der über ein Folienstück (14) mit mindestens einer an dem Folienstück (14) angeordneten elektrischen Leitung verfügt sowie über einen mit dieser elektrisch leitend verbundenen, bevorzugt an dem Folienstück (14) angeordneten Messfühler (13), wobei der Foliensensor (12) des Sensorteils (11) mit der Elektronikeinheit des Elektronikteils (17) der Sensoreinheit betreibbar und wobei das Sensorteil (11) bevorzugt lösbar mit dem Elektronikteil (17) verbindbar ist.
27. Sensorteil gemäß Ausführungsbeispiel 26, **weiter gekennzeichnet durch** ein oder mehrere Merkmale der Ausführungsbeispiele 1-25.
28. Elektronikteil für eine Sensoreinheit gemäß einem oder mehreren der vorhergehenden Ausführungsbeispiele, mit einer Elektronikeinheit zum Betreiben des Foliensensors (12) des Sensorteils (11) der Sensoreinheit, wobei das Elektronikteil (17) bevorzugt lösbar mit dem Sensorteil (11) verbindbar ist.
29. Elektronikteil gemäß Ausführungsbeispiel 28, **weiter gekennzeichnet durch** ein oder mehrere Merkmale der Ausführungsbeispiele 1-25.

**Bezugszeichenliste:**

| | | | |
|---|---|---|---|
| 10 | Sensoreinheit | 30 | Vertiefung |
| 11 | Sensorteil | 31 | Bodenwand |
| 12 | Foliensensor | 32 | Seitenwand |
| 13 | Messfühler | 33 | Durchtrittsöffnung |
| 14 | Folienstück | 34 | Messelektronikeinheit |
| 14a | erster Abschnitt Folienstück | 35 | Sendeeinheit |
| 14b | zweiter Abschnitt Folienstück | 36 | Energieversorgungseinheit |
| 14c | Verbindungsabschnitt Folienstück | 37 | Kamera |
| 15 | elektrische Leitungen | 38 | Erhebungen |
| 16 | Kontaktelemente Foliensensor | 39a | Film |
| 17 | Elektronikteil | 39b | Film |
| 18 | Elektronikeinheit | 40a | Film |
| 19 | Kontaktelemente Elektronikeinheit | 40b | Film |
| 20 | Leiterplatte | | |
| 21 | Klebematerialstück | | |
| 22 | elektrisch leitendes Klebematerialstück | | |
| 23 | Isolationsschicht | | |
| 24 | Schutzumhüllung | | |
| 25 | Materialstück Sensorteil | | |
| 26 | Umschlagbereich | | |
| 27 | Kante | | |
| 28 | Kantenbereich | | |
| 29 | Materialstück Elektronikteil | | |

## Patentansprüche

1. Sensoreinheit zur Befestigung an einem menschlichen oder tierischen Körper, insbesondere an der Haut des Körpers, mit einem Sensorteil (11), das einen Foliensensor (12) zur Messung ein oder mehrerer Körperparameter aufweist, der über ein Folienstück (14) mit mindestens einer an dem Folienstück (14) angeordneten elektrischen Leitung verfügt sowie über einen mit dieser Leitung elektrisch leitend verbundenen, bevorzugt an dem Folienstück (14) angeordneten Messfühler (13), und mit einem mit dem Sensorteil (11) bevorzugt lösbar verbundenen Elektronikteil (17), das eine Elektronikeinheit zum Betreiben des Foliensensors aufweist.

2. Sensoreinheit gemäß Anspruch 1, **dadurch gekennzeichnet, dass** das Elektronikteil (17) und/oder das Sensorteil (11) mindestens ein insbesondere elastisches Materialstück (25, 29) bevorzugt aus einem Schaummaterial aus Kunststoff, insbesondere aus Polyurethan, oder einem Elastomermaterial oder einem Silikon aufweist.

3. Sensoreinheit gemäß Anspruch 2, **dadurch gekennzeichnet, dass** die Elektronikeinheit teilweise oder vollständig in das Materialstück (29) des Elektronikteils (17) integriert oder eingebettet ist.

4. Sensoreinheit gemäß einem oder mehreren der vorhergehenden Ansprüche, mindestens gemäß Anspruch 2, **dadurch gekennzeichnet, dass** das Materialstück (25) des Sensorteils (11) eine Vertiefung (30) aufweist, und dass das Elektronikteil (17) in der Vertiefung (30) angeordnet ist, insbesondere unter lösbarer Verbindung des Elektronikteils (17), insbesondere des Materialstücks (29) desselben, mit dem Materialstück (25) des Sensorteils (11) vorzugsweise durch das doppelseitig klebende Klebematerialstück.

5. Sensoreinheit gemäß einem oder mehreren der vorhergehenden Ansprüche, **dadurch gekennzeichnet, dass** die Vertiefung (30) des Materialstücks (25) des Sensorteils (11) durch eine durch das Materialstück (25) gebildete Bodenwand begrenzt ist sowie durch eine mit dieser Bodenwand einstückig verbundene, durch das Materialstück (25) des Sensorteils (11) gebildete, die Vertiefung (30) seitlich umgebende Seitenwand.

6. Sensoreinheit gemäß einem oder mehreren der vorhergehenden Ansprüche, **dadurch gekennzeichnet, dass** das Materialstück (29) des Elektronikteils (17) die Vertiefung (30) des Sensorteils (11) zu einer (ansonsten offenen) Seite hin verschließt, insbesondere zu einer Seite, die der zu der Vertiefung (30) zeigenden Seite der Bodenwand gegenüberliegt.

7. Sensoreinheit gemäß einem oder mehreren der vorhergehenden Ansprüche, **dadurch gekennzeichnet, dass** das Materialstück (29) des Elektronikteils (17) kraftschlüssig mit der die Vertiefung (30) seitlich umgebenden Seitenwand verbunden ist, vorzugsweise, indem das Materialstück (29) des Elektronikteils (17) unter kraftschlüssiger Anlage an die Seitenwand mit (seitlichem) Übermaß in die Vertiefung (30) eingebracht ist.

8. Sensoreinheit gemäß einem oder mehreren der vorhergehenden Ansprüche, **dadurch gekennzeichnet, dass** ein erster Abschnitt des Folienstücks (14) des Foliensensors (12) einer ersten Seite des Materialstücks (25) des Sensorteils (11) zugeordnet ist und insbesondere mit dieser (bevorzugt unlösbar) verbunden ist, insbesondere der der Vertiefung (30) zugewandten Innenseite der Bodenwand, und dass ein zweiter Abschnitt des Folienstücks (14) des Foliensensors (12), einer, - bei bestimmungsgemäßer Verwendung der Sensoreinheit - dem Körper zugewandten, zweiten Seite des Materialstücks (25) des Sensorteils (11) zugeordnet und insbesondere mit dieser (bevorzugt unlösbar) verbunden ist, insbesondere der der Innenseite vorzugsweise parallel gegenüberliegenden Außenseite der Bodenwand.

9. Sensoreinheit gemäß Anspruch 8, **dadurch gekennzeichnet, dass** die beiden Abschnitte des Folienstücks (14) durch einen Verbindungsabschnitt des Folienstücks (14) insbesondere einstückig miteinander verbunden sind, der unter Ausbildung eines Umschlagbereichs, in dem der Verbindungsabschnitt insbesondere um 180°, bevorzugt u-förmig, um eine Kante eines vorzugsweise plattenförmigen Kantenbereichs des Materialstücks (25) des Sensorteils (11) umgeschlagen ist, von der ersten Seite zur zweiten Seite des Materialstücks (25) des Sensorteils (11) geführt ist.

10. Sensoreinheit gemäß einem oder mehreren der vorhergehenden Ansprüche, **dadurch gekennzeichnet, dass** der Messfühler (13) des Foliensensors (12) an einer - bei bestimmungsgemäßer Verwendung der Sensoreinheit - dem Körper zugewandten Außenseite des Sensorteils (11) angeordnet ist, insbesondere des Materialstücks (25) des Sensorteils (11), vorzugsweise an der Außenseite der die Vertiefung (30) begrenzenden Bodenwand des Sensorteils (11).

11. Sensoreinheit gemäß einem oder mehreren der vorhergehenden Ansprüche, **dadurch gekennzeichnet, dass** die Elektronikeinheit des Elektronikteils (17) eine insbesondere flexible Leiterplatte (20) mit ein oder mehreren elektronischen Bauteilen aufweist, insbesondere mit ein oder mehreren Messelektronikbauteilen und/oder einer Energieversorgungseinheit, insbesondere einer Batterie, zum Betreiben der Elektronikeinheit (17) und/oder einer Sende- und/oder Empfangseinheit zum drahtlosen Übertragen von Informationen an eine entfernte Sende- und/oder Empfangseinheit.

12. Sensoreinheit gemäß einem oder mehreren der vorhergehenden Ansprüche, **dadurch gekennzeichnet, dass** die Vertiefung (30) des Materialstücks (25) des Sensorteils (11) unter Bildung der Bodenwand und der Seitenwand durch Prägung in das Materialstück eingebracht ist.

13. Sensoreinheit gemäß einem oder mehreren der vorhergehenden Ansprüche, **dadurch gekennzeichnet, dass** der Foliensensor (14), insbesondere der der zweiten Seite des Materialstücks (25) des Sensorteils (11) zugeordnete zweite Abschnitt des Folienstücks des Foliensensors (12), ein oder mehrere Messfühler (13) aufweist, nämlich mindestens einen Temperatursensor und/oder einen Leitfähigkeitssensor und/oder einen Drucksensor und/oder einen Impedanzsensor.

14. Sensoreinheit gemäß einem oder mehreren der vorhergehenden Ansprüche, **dadurch gekennzeichnet, dass** die zweite Seite des Materialstücks (25) des Sensorteils (11) ein oder mehrere, gegenüber mindestens einem anderen, insbesondere ebenen Bereich dieser zweiten Seite hervorstehende Erhebungen (38) aufweist, und dass der oder jeder Messfühler (13) auf einer solchen Erhebung (38) angeordnet ist.

15. Sensorteil für eine Sensoreinheit gemäß einem oder mehreren der vorhergehenden Ansprüche, das einen Foliensensor (12) zur Messung mindestens eines Körperparameters aufweist, der über ein Folienstück (14) mit mindestens einer an dem Folienstück (14) angeordneten elektrischen Leitung verfügt sowie über einen mit dieser elektrisch leitend verbundenen, bevorzugt an dem Folienstück (14) angeordneten Messfühler (13), wobei der Foliensensor (12) des Sensorteils (11) mit der Elektronikeinheit des Elektronikteils (17) der Sensoreinheit betreibbar und wobei das Sensorteil (11) bevorzugt lösbar mit dem Elektronikteil (17) verbindbar ist.

16. Elektronikteil für eine Sensoreinheit gemäß einem oder mehreren der vorhergehenden Ansprüche, mit einer Elektronikeinheit zum Betreiben des Foliensensors (12) des Sensorteils (11) der Sensoreinheit, wobei das Elektronikteil (17) bevorzugt lösbar mit dem Sensorteil (11) verbindbar ist.
